# EUROPEAN PATENT SPECIFICATION

(11) **EP 4 536 142 B1**
(45) Date of publication and mention of the grant of the patent: **02.12.2026**
(21) Application number: 23732685.5
(22) Date of filing: 07.06.2023
(51) Int. Cl.: A61F 2/24, A61M 25/01

(54) **TRANSLUMINAL DELIVERY SYSTEM**
TRANSLUMINALES ABGABESYSTEM
SYSTÈME DE MISE EN PLACE TRANSLUMINALE

(30) Priority: 08.06.2022 US 202263350200 P
(43) Date of publication of application: 16.04.2025
(73) Proprietor: Cardiovalve Ltd., 6037501 Or Yehuda (IL)
(72) Inventor: ALBITOV, Michael, 5545119 Kiryat Ono (IL); KARALNIK, Maxim, 2198716 Karmiel (IL); IAMBERGER, Meni, 4464621 Kfar Saba (IL); HARITON, Ilia, 3091888 Zichron Yaakov (IL); HAMMER, Tal, 5224213 Ramat Gan (IL)
(74) Representative: Evens, Paul Jonathan
(86) International application number: PCT/IL2023/050586
(87) International publication number: WO 2023/238129

(56) References cited:
- US-A1- 2011 257 733
- US-A1- 2018 153 695
- US-A1- 2019 083 262

## Description

### FIELD OF THE INVENTION

The present invention relates to transluminal implant-delivery systems. The present disclosure relates to prosthetic heart valves, and transluminal delivery systems therefor.

### BACKGROUND

Dilation of the annulus of a heart valve, such as that caused by ischemic heart disease, prevents the valve leaflets from fully coapting when the valve is closed. Regurgitation of blood from the ventricle into the atrium results in increased total stroke volume and decreased cardiac output, and ultimate weakening of the ventricle secondary to a volume overload and a pressure overload of the atrium.

### SUMMARY OF THE INVENTION

The present invention is defined in claim 1 and is directed towards an apparatus for delivering an implant to a subject.

For some applications, there is provided, in accordance with the present disclosure, an apparatus for use at a heart of a subject, the apparatus including:
a delivery tool dimensioned for percutaneous delivery of an implant to the heart, the delivery tool including:
a catheter defining a catheter-lumen,
a shaft, at least a portion of the shaft slidably housed within the catheter-lumen, and
a distal portion that defines a distal portion axis and includes:
   a capsule, the capsule:
      configured to house at least a portion of the implant,
      coupled to a distal portion of the shaft, and
      slidable along the distal portion axis, with respect to the catheter, so that the capsule transitions from:
         a first state in which the capsule is disposed at a first distance from a distal end of the catheter, to
         a second state in which the capsule is disposed at a second distance from the distal end of the catheter,
      the second distance is greater than the first distance; and
   a support, the support:
      fixedly coupled to a distal region of the catheter,
      extending:
         along the distal portion axis, and
         from the distal end of the catheter toward the capsule, and
      configured to expand, while the capsule transitions from the first state to the second state:
         from a contracted state in which the support has a first length, to
         an expanded state in which the support has a second length.

In an application, a proximal region of the support is fixedly coupled to the distal region of the catheter.

In an application:
the support is configured to define a support-lumen,
a portion of the shaft is disposed within the support-lumen, and
while the support is in the expanded state, the support has a stiffness that is greater than a stiffness of the portion of the shaft.

In an application, the support is configured such that while the capsule transitions from the first state to the second state, the support self-expands from the contracted state to the expanded state.

In an application:
the capsule is reversibly slidable along the distal portion axis, with respect to the catheter, such that the capsule is transitionable from the second state to the first state, and
while the capsule transitions from the second state to the first state, the support is configured to contract from the expanded state to the contracted state.

In an application, while the support is in the contracted state, the support has a radial thickness of 1 - 3 mm.

In an application, while the support is in the contracted state, the support has a length of 4 - 18 mm.

In an application, while the support is in the expanded state, the support has an expanded length that is between 10 mm and 60 mm.

In an application:
while the support is in the contracted state, the support has a contracted length, and
the expanded length that is 10 - 50 mm longer than the contracted length.

In an application:
while the support is in the contracted state, the support has a contracted length, and
the expanded length is 2 - 5 times longer than the contracted length.

In an application, the support is configured such that while the support is in the expanded state, the support extends from the distal end of the catheter to the capsule.

In an application, the support is configured such that while the support expands from the contracted state to the expanded state, the support extends from the distal end of the catheter to the capsule.

In an application:
the support includes a plurality of coaxial segments,
a proximal one of the coaxial segments is coupled to the distal region of the catheter, and
the support is configured such that, while the support expands from the contracted state to the expanded state, at least one of the segments slides distally from the proximal segment.

In an application, a distal segment is fixedly coupled to the capsule.

In an application, the plurality of coaxial segments includes at least 2 segments.

In an application, the plurality of coaxial segments includes fewer than 15 segments.

In an application, the plurality of coaxial segments includes 2 - 15 segments.

In an application, at least two of the segments have a radial thickness of 0.05 - 0.5 mm.

In an application, at least two of the segments have a length of 3 - 15 mm.

In an application, the support is configured such that, while the support expands from the contracted state to the expanded state, a first one of the segments that is adjacent to a second one of the segments slides distally, along the distal portion axis, between 2 mm and 14 mm with respect to the second one of the segments.

In an application:
each of the segments longitudinally overlaps with at least one other of the segments, and
while the support is in the expanded state, at least two of the segments longitudinally overlap to a lesser degree than while the support is in the contracted state.

In an application, while the support is in the expanded state, a diameter of a distal-most segment is 2 - 10 mm greater than a diameter of the proximal segment.

In an application, while the support is in the expanded state, a largest dimension of a distal-most segment perpendicular to the distal portion axis is 2 - 10 mm greater than a largest dimension perpendicular to the distal portion axis of the proximal segment.

The present invention will be more fully understood from the following detailed description of applications thereof, taken together with the drawings, in which:

### BRIEF DESCRIPTION OF THE DRAWINGS

Figs. 1A-B are schematic illustrations showing a catheter support for use with a catheter, in accordance with some applications of the invention; and
Figs. 2A-B are schematic illustrations showing use of a distal portion of a delivery tool, in accordance with some applications of the invention.

### DETAILED DESCRIPTION OF EMBODIMENTS

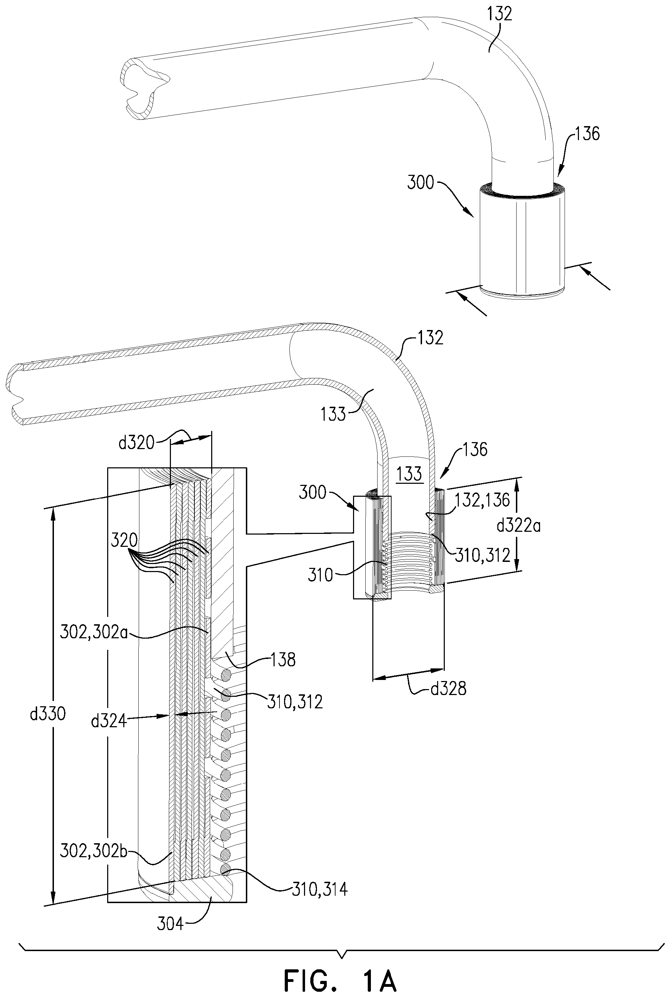

Reference is made to Figs. 1A-B, which are schematic illustrations showing a catheter support 300 for use with a catheter 132, in accordance with some applications of the invention. Support 300 may be used with other catheters.

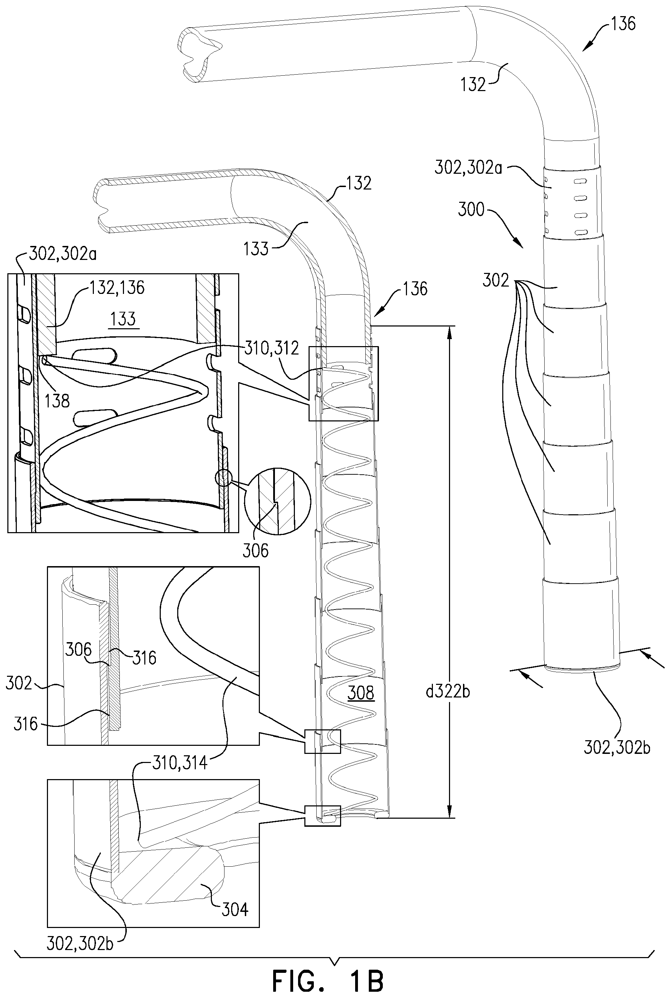

As shown, support 300 has a contracted state (Fig. 1A) and an expanded state (Fig. 1B). As shown in Fig. 1B, the support is shaped to define a support-lumen 308, and is typically used to mechanically support elements that are disposed within and beyond the support-lumen. For the sake of clarity, Figs. 1A-B show support 300 without an element disposed within support-lumen 308.

Support 300 is fixedly coupled to a distal region 136 of catheter 132, and it is typically desirable for the support to have a limited profile when it is in the contracted state, in order for the contracted support to add as little bulk as possible to the contour of the catheter. For some applications, while support 300 is in the contracted state, the support has a radial thickness d320 of between 1 mm and 3 mm.

It is also typically desirable that support 300 has a limited length while it is in the contracted state. For some applications, and as shown, support 300 comprises a plurality of coaxial segments 302 that are nested with respect to each other. Typically for such applications, support 300 has at least two segments. For example, support 300 may have fewer than 15 segments, e.g., 2-15 segments. Segments 302 are typically dimensioned to each have a limited length d330 (e.g., between 4 mm and 18 mm) and a radial thickness d324 (e.g., between 0.05 mm and 0.5 mm), thereby reducing the total length and thickness of support 300 while the support is contracted.

Segments 302 typically longitudinally overlap while support 300 is contracted (Fig. 1A), further reducing the support's total length d322a when the support is contracted. Typically for such applications, support 300 is expanded by reducing the longitudinal overlap between segments 302, e.g., at least some segments 302 move longitudinally with respect to others of the segments 302 in order for support 300 to expand.

For some applications, support 300 has an expanded length d322b (Fig. 1B) that is two to five times longer than contracted length d322a. For example, support 300 may expand to become between 10 and 60 mm longer (e.g., between 10 and 50 mm longer) than contracted length d322a (Fig. 1A).

For some applications, in order for support 300 to expand, at least one of the segments slides distally from proximal segment 302a. For example, and as shown, segments 302 move distally with respect to segments having a smaller diameter. Since each progressively distal segment 302 has a larger diameter than the immediately proximal segment, support 300 tapers proximally in the expanded state. For some such applications, while the support is in the expanded state, a diameter of distal segment 302b is between 2-10 mm greater than a diameter of proximal segment 302a.

For some applications, in the absence of an external force being applied, support 300 self-expands from the contracted state to the expanded state, e.g., due to a spring 310 disposed within support 300 being in compression when support 300 is in the contracted state. Typically for such applications, and as shown, spring 310 occupies only a portion of support-lumen 308, such that unoccupied space remains within the support-lumen for passage of elements that support 300 mechanically supports. Typically, a proximal portion 312 of the spring contacts a distal region 136 (e.g., distal end 138) of catheter 132, and a distal portion 314 of the spring contacts a distal portion 304 of the support. For some applications, and as shown in the inset of Fig. 1A, proximal segment 302a is shaped to define grooves in which a portion of spring 310 is disposed.

For some such applications, and as shown, the self-expansion of support 300 is limited, such that the support does not expand beyond the expanded length. For example, and as shown in the circular inset of Fig. 1B, each pair of segments 302 may be complimentarily shaped to define a step 316. When segments 302 slide progressively distally, each successive segment 302 is pulled distally, until respective steps 316 of each pair of segments meet each other at an interface 306, thereby inhibiting further longitudinal expansion of support 300.

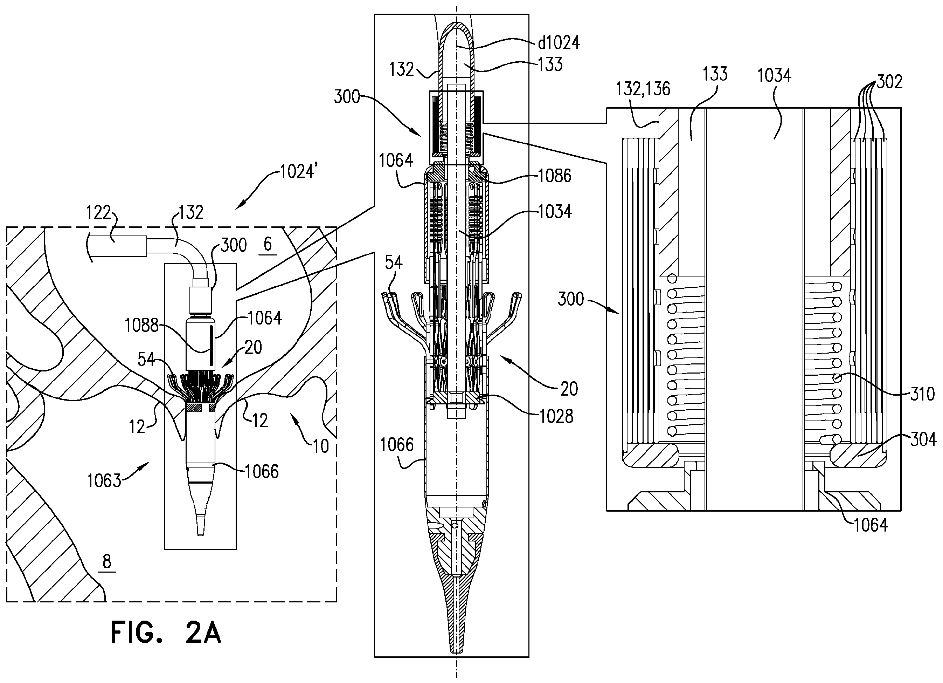

Reference is made to Figs. 2A-B, which are schematic illustrations showing use of a distal portion 1024' of a delivery tool, in accordance with some applications of the invention.

Distal portion 1024' is shown in Figs. 2A-B being used to deliver an implant 20 (e.g., a prosthetic valve) at the mitral valve. A capsule assembly 1063 is shown being delivered to the mitral valve using a first catheter 122 and a second catheter 132 of a steerable catheter system. This nonlimiting example is not meant to exclude use of other steerable catheter systems in combination with the apparatus described hereinabove with reference to Figs. 1A-B, *mutatis mutandis.*

Fig. 2A shows distal portion 1024' being used to deliver implant 20 to the mitral valve, at a stage of deployment in which a plurality of flanges 54 have been deployed within atrium 6. The cross-sectional view of Fig. 2A shows a shaft 1034 extending from within a catheter-lumen 133 of catheter 132, through support 300 and to a mount 1028. Typically, at least a portion of shaft 1034 has a greater flexibility than catheter 132. While the flexibility of shaft 1034 may be advantageous while the shaft is navigated through the confines of the vasculature, additional rigidity may be desired when advancing capsule assembly 1063 into ventricle 8. That is, the flexibility of shaft 1034 could allow mechanical forces that capsule assembly 1063 may encounter when advancing through the mitral valve to alter an orientation of the capsule assembly. As described below, support 300 is configured to reduce the likelihood that such mechanical forces would alter an orientation of capsule assembly 1063.

In the right inset of Fig. 2A, support 300 is in the contracted state, and distal portion 304 of support 300 is shown abutting a proximal capsule 1064 of capsule assembly 1063. While support 300 is in the contracted state, spring 310 is compressed, such that it applies a pushing force to proximal capsule 1064. The operator regulates the distal advancement of capsule assembly 1063, by controlling the advancement of shaft 1034, to which the capsule assembly is coupled.

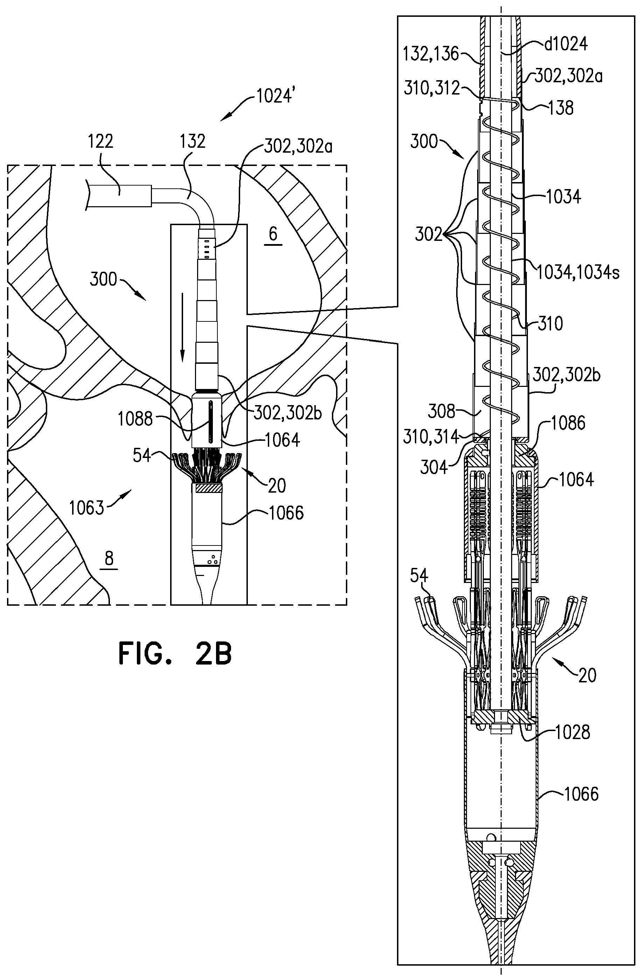

Fig. 2B shows implant 20 at a stage of deployment at the mitral valve in which the capsule assembly is advanced distally, through the mitral valve, e.g., until it is determined that leaflets 12 of a native valve 10 are coapting upstream of flanges 54 during ventricular systole. As shown in Fig. 2B, distally advancing capsule assembly 1063 along a distal portion axis d1024, by distally advancing shaft 1034, allows support 300 to self-expand into the expanded state. Since support 300 has a greater stiffness than shaft 1034, the expanded support provides mechanical support to a supported portion 1034s of shaft 1034 that is disposed within support-lumen 308, thereby stabilizing the capsule assembly and/or implant 20 that are disposed distally to support-lumen 308. For some applications, and as shown, support 300 (e.g., distal portion 304 thereof) continues to extend to capsule assembly 1063 while the support is in the expanded state. For some such applications, support 300 (e.g., distal segment 302b thereof) is fixedly coupled to capsule assembly 1063 (e.g., to proximal capsule 1064).

In this way, upon suitable orienting of the capsule assembly, support 300 facilitates placement of the flanges 54 downstream of the leaflets, as shown in Fig. 2B, by reducing the effect of mechanical forces that capsule assembly 1063 may encounter when advancing through the mitral valve.

Subsequently, capsule assembly 1063 is typically retracted proximally (e.g., along distal portion axis d1024), with respect to catheter 132, until it is determined that flanges 54 have engaged leaflets 12. Typically for such applications, proximal movement of capsule assembly 1063 causes support 300 to contract at least partially from the expanded state to the contracted state, e.g., by proximal capsule 1064 pushing proximally upon distal portion 304 of the support. That is, support 300 typically does not include a locking mechanism that would prevent proximal movement of the capsule assembly from contracting the support. After capsule assembly 1063 has been partially retracted as described, proximal capsule 1064 is typically retracted proximally, in relation to shaft 1034, further exposing the implant.

For some applications, proximal capsule 1064 is proximally retracted by applying a rotational force to a proximal end of shaft 1034 or other longitudinal member that engages a disc-assembly 1086. Disc-assembly 1086 is typically fitted within proximal capsule 1064, and comprises a proximal disc that is rotationally coupled to and rotationally movable with respect to a distal disc. Further typically, the exterior of the proximal disc defines external screw threading that is complementary to internal screw threading defined by the interior of proximal capsule 1064. Further typically for such applications, the proximal capsule 1064 is shaped to define one or more longitudinal tracks 1088 that traverses internal threading. Since the proximal disc is fixedly coupled to the capsule catheter, rotation of the capsule catheter with respect to shaft 1034 screws the proximal disc along internal threading of proximal capsule 1064. At the same time, the distal disc is inhibited from rotating because distal disc: (i) is fixedly coupled to shaft 1034, and (2) engages track 1088 of proximal capsule 1064. Thus, screwing of the proximal disc pushes the distal disc along track 1088, thereby translating rotational movement of the proximal disc into axial movement of proximal capsule 1064 with respect to disc-assembly 1086, as well as to mount 1028. Finally, distal capsule 1066 is advanced distally in order to further expose the implant and mount 1028, after which distal portion 1024' is withdrawn from the subject. Further details regarding this technique are described, mutatis mutandis, with reference to Fig. 14D of US Provisional Patent Application 63/350,200 to Albitov et al.

## Claims

1. Apparatus for use at a heart of a subject, the apparatus comprising:
a delivery tool dimensioned for percutaneous delivery of an implant to the heart, the delivery tool comprising:
a catheter (132) defining a catheter-lumen (133),
a shaft (1034), at least a first portion of the shaft (1034) slidably housed within the catheter-lumen (133), and
a distal portion (1024') that defines a distal portion axis (d1024) and comprises:
a capsule (1064,1066), the capsule (1064,1066):
configured to house at least a portion of the implant,
coupled to a distal portion of the shaft (1034), and
slidable along the distal portion axis (d1024), with respect to the catheter (132), so that the capsule (1064,1066) transitions from:
a first state in which the capsule (1064,1066) is disposed at a first distance from a distal end (138) of the catheter (132), to
a second state in which the capsule (1064,1066) is disposed at a second distance from the distal end (138) of the catheter 132),
wherein the second distance is greater than the first distance;
**characterized in that** the distal portion (1024') of the delivery tool further comprises a support (300), the support (300):
fixedly coupled to a distal region (136) of the catheter (132),
extending:
along the distal portion axis (d1024), and
from the distal end (138) of the catheter (132) toward the capsule (1064,1066), and
configured to expand, while the capsule (1064,1066) transitions from the first state to the second state:
from a contracted state in which the support (300) has a first length, to
an expanded state in which the support (300) has a second length.

2. The apparatus according to claim 1, wherein a proximal region (302a) of the support (300) is fixedly coupled to the distal region (136) of the catheter (132).

3. The apparatus according to claim 1, wherein:
the support (300) is configured to define a support-lumen (308),
a second portion of the shaft (1034) is disposed within the support-lumen (308), and
while the support (300) is in the expanded state, the support (300) has a stiffness that is greater than a stiffness of the second portion of the shaft (1034).

4. The apparatus according to claim 1, wherein the support (300) is configured such that while the capsule (1064,1066) transitions from the first state to the second state, the support (300) self-expands from the contracted state to the expanded state.

5. The apparatus according to claim 1, wherein:
the capsule (1064,1066) is reversibly slidable along the distal portion axis (d1024), with respect to the catheter (132), such that the capsule (1064,1066) is transitionable from the second state to the first state, and
while the capsule (1064,1066) transitions from the second state to the first state, the support (300) is configured to contract from the expanded state to the contracted state.

6. The apparatus according to any one of claims 1-5, wherein:
while the support (300) is in the contracted state, the support (300) has a contracted length, and
the expanded length that is 10 - 50 mm longer than the contracted length.

7. The apparatus according to any one of claims 1-5, wherein:
while the support (300) is in the contracted state, the support has a contracted length, and
the expanded length is 2 - 5 times longer than the contracted length.

8. The apparatus according to any one of claims 1-5, wherein:
the support (300) comprises a plurality of coaxial segments (302),
a proximal one (302a) of the coaxial segments (302) is coupled to the distal region (136) of the catheter (132), and
the support (300) is configured such that, while the support (300) expands from the contracted state to the expanded state, at least one of the segments (302) slides distally from the proximal segment (302a).

9. The apparatus according to claim 8, wherein a distal segment (302b) is fixedly coupled to the capsule (1064,1066).

10. The apparatus according to claim 8, wherein the plurality of coaxial segments (302) comprises 2 - 15 segments.

11. The apparatus according to claim 8, wherein at least two of the segments (302) have a length of 3 - 15 mm.

12. The apparatus according to claim 8, wherein, the support (300) is configured such that, while the support (300) expands from the contracted state to the expanded state, a first one of the segments (302) that is adjacent to a second one of the segments (302) slides distally, along the distal portion axis (d1024), between 2 mm and 14 mm with respect to the second one of the segments (302).

13. The apparatus according to claim 8, wherein:
each of the segments (302) longitudinally overlaps with at least one other of the segments (302), and
while the support (300) is in the expanded state, at least two of the segments (302) longitudinally overlap to a lesser degree than while the support (300) is in the contracted state.

14. The apparatus according to claim 13, wherein, while the support (300) is in the expanded state, a diameter of a distal-most segment (302b) is 2 - 10 mm greater than a diameter of the proximal segment (302a).

15. The apparatus according to claim 13, wherein, while the support (300) is in the expanded state, a largest dimension of a distal-most segment (302b) perpendicular to the distal portion axis (d1024) is 2 - 10 mm greater than a largest dimension perpendicular to the distal portion axis (d1024) of the proximal segment (302a).

## Patentansprüche

1. Vorrichtung zur Verwendung an einem Herzen eines Subjekts, wobei die Vorrichtung Folgendes umfasst:
ein Abgabewerkzeug, das für die perkutane Abgabe eines Implantats an das Herz dimensioniert ist, wobei das Abgabewerkzeug Folgendes umfasst:
einen Katheter (132), der ein Katheterlumen (133) definiert,
einen Schaft (1034), wobei mindestens ein erster Abschnitt des Schafts (1034) verschiebbar innerhalb des Katheterlumens (133) untergebracht ist, und
einen distalen Abschnitt (1024'), der eine distale Abschnittsachse (d1024) definiert und Folgendes umfasst:
eine Kapsel (1064, 1066), wobei die Kapsel (1064, 1066):
dazu konfiguriert ist, mindestens einen Abschnitt des Implantats unterzubringen,
an einen distalen Abschnitt des Schafts (1034) gekoppelt ist, und
in Bezug auf den Katheter (132) entlang der distalen Abschnittsachse (d1024) verschiebbar ist, sodass die Kapsel (1064, 1066) übergeht von:
einem ersten Zustand, in dem die Kapsel (1064, 1066) in einem ersten Abstand von einem distalen Ende (138) des Katheters (132) angeordnet ist, in
einen zweiten Zustand, in dem die Kapsel (1064, 1066) in einem zweiten Abstand von dem distalen Ende (138) des Katheters 132) angeordnet ist,
wobei der zweite Abstand größer als der erste Abstand ist;
**dadurch gekennzeichnet, dass** der distale Abschnitt (1024') des Abgabewerkzeugs ferner eine Stütze (300) umfasst, wobei die Stütze (300):
fest an eine distale Region (136) des Katheters (132) gekoppelt ist, der sich erstreckt:
entlang der distalen Abschnittsachse (d1024) und
von dem distalen Ende (138) des Katheters (132) zu der Kapsel (1064, 1066) hin und
dazu konfiguriert ist, sich wie folgt zu erweitern, während die Kapsel (1064, 1066) von dem ersten Zustand in den zweiten Zustand übergeht:
von einem zusammengezogenen Zustand, in dem die Stütze (300) eine erste Länge aufweist, zu
einem erweiterten Zustand, in dem die Stütze (300) eine zweite Länge aufweist.

2. Vorrichtung nach Anspruch 1, wobei eine proximale Region (302a) der Stütze (300) fest an die distale Region (136) des Katheters (132) gekoppelt ist.

3. Vorrichtung nach Anspruch 1, wobei:
die Stütze (300) dazu konfiguriert ist, ein Stützenlumen (308) zu definieren,
ein zweiter Abschnitt des Schafts (1034) innerhalb des Stützenlumens (308) angeordnet ist, und
während sich die Stütze (300) in dem erweiterten Zustand befindet, die Stütze (300) eine Steifigkeit aufweist, die größer als eine Steifigkeit des zweiten Abschnitts des Schafts (1034) ist.

4. Vorrichtung nach Anspruch 1, wobei die Stütze (300) so konfiguriert ist, dass, während die Kapsel (1064, 1066) von dem ersten Zustand in den zweiten Zustand übergeht, sich die Stütze (300) selbst von dem zusammengezogenen Zustand zu dem erweiterten Zustand erweitert.

5. Vorrichtung nach Anspruch 1, wobei:
die Kapsel (1064, 1066) in Bezug auf den Katheter (132) entlang der distalen Abschnittsachse (d1024) reversibel verschiebbar ist, sodass die Kapsel (1064, 1066) von dem zweiten Zustand in den ersten Zustand überführbar ist, und
während die Kapsel (1064, 1066) von dem zweiten Zustand in den ersten Zustand übergeht, die Stütze (300) dazu konfiguriert ist, sich von dem erweiterten Zustand zu dem zusammengezogenen Zustand zusammenzuziehen.

6. Vorrichtung nach einem der Ansprüche 1-5, wobei:
während sich die Stütze (300) in dem zusammengezogenen Zustand befindet, die Stütze (300) eine zusammengezogene Länge aufweist, und
die erweiterte Länge, die 10 - 50 mm länger als die zusammengezogene Länge ist.

7. Vorrichtung nach einem der Ansprüche 1-5, wobei:
während sich die Stütze (300) in dem zusammengezogenen Zustand befindet, die Stütze eine zusammengezogene Länge aufweist, und
die erweiterte Länge 2-5-mal länger als die zusammengezogene Länge ist.

8. Vorrichtung nach einem der Ansprüche 1-5, wobei:
die Stütze (300) eine Vielzahl von koaxialen Segmenten (302) umfasst,
ein proximales (302a) der koaxialen Segmente (302) an die distale Region (136) des Katheters (132) gekoppelt ist und
die Stütze (300) so konfiguriert ist, dass, während sich die Stütze (300) von dem zusammengezogenen Zustand zu dem erweiterten Zustand erweitert, mindestens eines der Segmente (302) sich distal von dem proximalen Segment (302a) verschiebt.

9. Vorrichtung nach Anspruch 8, wobei ein distales Segment (302b) fest an die Kapsel (1064, 1066) gekoppelt ist.

10. Vorrichtung nach Anspruch 8, wobei die Vielzahl von koaxialen Segmenten (302) 2 - 15 Segmente umfasst.

11. Vorrichtung nach Anspruch 8, wobei mindestens zwei der Segmente (302) eine Länge von 3 - 15 mm aufweisen.

12. Vorrichtung nach Anspruch 8, wobei die Stütze (300) so konfiguriert ist, dass, während sich die Stütze (300) von dem zusammengezogenen Zustand zu dem erweiterten Zustand erweitert, ein erstes der Segmente (302), das benachbart zu einem zweiten der Segmente (302) ist, sich distal entlang der distalen Abschnittsachse (d1024) zwischen 2 mm und 14 mm in Bezug auf das zweite der Segmente (302) verschiebt.

13. Vorrichtung nach Anspruch 8, wobei:
jedes der Segmente (302) sich in Längsrichtung mit mindestens einem anderen der Segmente (302) überlappt, und
während sich die Stütze (300) in dem erweiterten Zustand befindet, sich mindestens zwei der Segmente (302) in Längsrichtung in einem geringeren Ausmaß überlappen, als während sich die Stütze (300) in dem zusammengezogenen Zustand befindet.

14. Vorrichtung nach Anspruch 13, wobei, während sich die Stütze (300) in dem erweiterten Zustand befindet, ein Durchmesser eines distalsten Segments (302b) 2 - 10 mm größer als ein Durchmesser des proximalen Segments (302a) ist.

15. Vorrichtung nach Anspruch 13, wobei, während sich die Stütze (300) in dem erweiterten Zustand befindet, eine größte Abmessung eines distalsten Segments (302b) senkrecht zu der distalen Abschnittsachse (d1024) 2 - 10 mm größer als eine größte Abmessung senkrecht zu der distalen Abschnittsachse (d1024) des proximalen Segments (302a) ist.

## Revendications

1. Appareil destiné à être utilisé au niveau du cœur d'un patient, l'appareil comprenant :
un outil de mise en place dimensionné pour une mise en place par voie percutanée d'un implant dans le cœur, l'outil de mise en place comprenant :
un cathéter (132) définissant une lumière de cathéter (133),
une tige (1034), au moins une première partie de la tige (1034) étant logée de manière coulissante à l'intérieur de la lumière de cathéter (133), et
une partie distale (1024') qui définit un axe de partie distale (d1024) et comprend :
une capsule (1064, 1066), la capsule (1064, 1066) étant :
conçue pour loger au moins une partie de l'implant,
couplée à une partie distale de la tige (1034), et
coulissante le long de l'axe de partie distale (d1024), par rapport au cathéter (132), de sorte que la capsule (1064, 1066) passe de :
un premier état dans lequel la capsule (1064, 1066) est disposée à une première distance d'une extrémité distale (138) du cathéter (132), à
un second état dans lequel la capsule (1064, 1066) est disposée à une seconde distance de l'extrémité distale (138) du cathéter 132),
dans lequel la seconde distance est supérieure à la première distance ;
**caractérisé en ce que** la partie distale (1024') de l'outil de mise en place comprend en outre un support (300), le support (300) :
couplé de manière fixe à une région distale (136) du cathéter (132),
s'étendant :
le long de l'axe de partie distale (d1024), et
de l'extrémité distale (138) du cathéter (132) vers la capsule (1064, 1066), et
configuré pour se déployer, tandis que la capsule (1064, 1066) passe du premier état au second état :
depuis un état contracté dans lequel le support (300) présente une première longueur, à
un état déployé dans lequel le support (300) présente une seconde longueur.

2. Appareil selon la revendication 1, dans lequel une région proximale (302a) du support (300) est couplée de manière fixe à la région distale (136) du cathéter (132).

3. Appareil selon la revendication 1, dans lequel :
le support (300) est conçu pour définir une lumière de support (308),
une seconde partie de la tige (1034) est disposée à l'intérieur de la lumière de support (308), et
tandis que le support (300) est dans l'état déployé, le support (300) présente une rigidité qui est supérieure à une rigidité de la seconde partie de tige (1034).

4. Appareil selon la revendication 1, dans lequel le support (300) est conçu de sorte que tandis que la capsule (1064, 1066) passe du premier état au second état, le support (300) se déploie automatiquement de l'état contracté à l'état déployé.

5. Appareil selon la revendication 1, dans lequel :
la capsule (1064, 1066) peut coulisser de manière réversible le long de l'axe de partie distale (d1024), par rapport au cathéter (132), de sorte que la capsule (1064, 1066) puisse passer du second état au premier état, et
tandis que la capsule (1064, 1066) passe du second état au premier état, le support (300) est conçu pour se contracter de l'état déployé à l'état contracté.

6. Appareil selon l'une quelconque des revendications 1 à 5, dans lequel :
tandis que le support (300) est dans l'état contracté, le support (300) présente une longueur contractée, et
la longueur déployée qui est de 10 à 50 mm plus longue que la longueur contractée.

7. Appareil selon l'une quelconque des revendications 1 à 5, dans lequel :
tandis que le support (300) est dans l'état contracté, le support présente une longueur contractée, et
la longueur déployée est de 2 à 5 fois plus longue que la longueur contractée.

8. Appareil selon l'une quelconque des revendications 1 à 5, dans lequel :
le support (300) comprend une pluralité de segments coaxiaux (302),
un segment proximal (302a) des segments coaxiaux (302) est couplé à la région distale (136) du cathéter (132), et
le support (300) est conçu de sorte que, tandis que le support (300) se déploie de l'état contracté à l'état déployé, au moins un des segments (302) coulisse de manière distale à partir du segment proximal (302a).

9. Appareil selon la revendication 8, dans lequel un segment distal (302b) est couplé de manière fixe à la capsule (1064, 1066).

10. Appareil selon la revendication 8, dans lequel la pluralité de segments coaxiaux (302) comprennent de 2 à 15 segments.

11. Appareil selon la revendication 8, dans lequel au moins deux des segments (302) ont une longueur de 3 à 15 mm.

12. Appareil selon la revendication 8, dans lequel le support (300) est conçu de sorte que, tandis que le support (300) se déploie de l'état contracté à l'état déployé, un premier des segments (302) qui est adjacent à un deuxième des segments (302) coulisse de manière distale, le long de l'axe de partie distale (d1024), entre 2 mm et 14 mm par rapport au deuxième des segments (302).

13. Appareil selon la revendication 8, dans lequel :
chacun des segments (302) chevauche longitudinalement au moins un autre des segments (302), et
tandis que le support (300) est dans l'état déployé, au moins deux des segments (302) se chevauchent longitudinalement à un degré moindre que lorsque le support (300) est dans l'état contracté.

14. Appareil selon la revendication 13, dans lequel, tandis que le support (300) est dans l'état déployé, un diamètre d'un segment le plus distal (302b) est supérieur de 2 à 10 mm à un diamètre du segment proximal (302a).

15. Appareil selon la revendication 13, dans lequel, tandis que le support (300) est dans l'état déployé, une dimension la plus grande d'un segment le plus distal (302b) perpendiculaire à l'axe de partie distale (d1024) est supérieure de 2 à 10 mm à une dimension la plus grande perpendiculaire à l'axe de partie distale (d1024) du segment proximal (302a).
